(19) Europäisches Patentamt
European Patent Office
Office européen des brevets

(11) Publication number: **0 337 549 B1**

(12) # EUROPEAN PATENT SPECIFICATION

(45) Date of publication of patent specification: **04.10.95**

(51) Int. Cl.$^6$: **C07D 205/08**, A61K 31/395

(21) Application number: **89200864.0**

(22) Date of filing: **06.04.89**

The file contains technical information submitted after the application was filed and not included in this specification

(54) **New substituted azetidinones as anti-inflammatory and antidegenerative agents.**

(30) Priority: **11.04.88 US 179688**

(43) Date of publication of application: **18.10.89 Bulletin 89/42**

(45) Publication of the grant of the patent: **04.10.95 Bulletin 95/40**

(84) Designated Contracting States: **AT BE CH DE ES FR GB GR IT LI LU NL SE**

(56) References cited: **EP-A- 0 199 630**

(73) Proprietor: **MERCK & CO. INC.**
**126, East Lincoln Avenue**
**P.O. Box 2000**
**Rahway**
**New Jersey 07065-0900 (US)**

(72) Inventor: **Shah, Shrenik K.**
**25 Denise Court**
**Metuchen**
**New Jersey 08840 (US)**
Inventor: **Finke, Paul E.**
**34 Inwood Drive**
**Milltown**
**New Jersey 08850 (US)**
Inventor: **Doherty, James B.**
**559 Columbia Street**
**New Milford**
**New Jersey 07646 (US)**
Inventor: **Barker, Peter L.**
**518 Hort Street**
**Westfield**
**New Jersey 07090 (US)**
Inventor: **Hagmann, William**
**309 Hyslip Avenue**
**Westfield**
**New Jersey 07090 (US)**
Inventor: **Dorn, Conrad P.**
**972 Fernwood Avenue**
**Plainfield**
**New Jersey 07062 (US)**
Inventor: **Firestone, Raymond A.**
**387 Temple Street**
**New Haven**
**Connecticut 06511 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 337 549 B1

(74) Representative: **Cole, William Gwyn**
**European Patent Department**
**Merck & Co., Inc.**
**Terlings Park**
**Eastwick Road**
**Harlow**
**Essex CM20 2QR (GB)**

## Description

### BACKGROUND OF THE INVENTION

We have found that a group of new substituted azetidinones are potent elastase inhibitors and therefore are useful antiinflammatory/antidegenerative agents.

Proteases from granulocytes and macrophages have been reported to be responsible for the chronic tissue destruction mechanisms associated with inflammation, including rheumatoid arthritis and emphysema. Accordingly, specific and selective inhibitors of these proteases are candidates for potent anti-inflammatory agents useful in the treatment of inflammatory conditions resulting in connective tissue destruction, e.g. rheumatoid arthritis, emphysema, bronchial inflammation, osteoarthritis, spondylitis, lupus, psoriasis, atherosclerosis, sepsis, septicemia, shock, periodontitis, cystic fibrosis and acute respiratory distress syndrome.

The role of proteases from granulocytes, leukocytes or macrophages are related to a rapid series of events which occurs during the progression of an inflammatory condition:

(1) There is a rapid production of prostaglandins (PG) and related compounds synthesized from arachidonic acid. This PG synthesis has been shown to be inhibited by aspirin-related nonsteroidal anti-inflammatory agents including indomethacin and phenylbutazone. There is some evidence that protease inhibitors prevent PG production;

(2) There is also a change in vascular permeability which causes a leakage of fluid into the inflamed site and the resulting edema is generally used as a marker for measuring the degree of inflammation. This process has been found to be induced by the proteolytic or peptide cleaving activity of proteases, especially those contained in the granulocyte, and thereby can be inhibited by various synthetic protease inhibitors, for example, N-acyl benzisothiazolones and the respective 1,1-dioxides. Morris Zimmerman et al., J. Biol. Chem., 255, 9848 (1980); and

(3) There is an appearance and/or presence of lymphoid cells, especially macrophages and polymorphonuclear leukocytes (PMN). It has been known that a variety of proteases are released from the macrophages and PMN, further indicating that the proteases do play an important role in inflammation.

In general, proteases are an important family of enzymes within the peptide bond cleaving enzymes whose members are essential to a variety of normal biological activities, such as digestion, formation and dissolution of blood clots, the formation of active forms of hormones, the immune reaction to foreign cells and organisms, etc., and in pathological conditions such as the degradation of structural proteins at the articular cartilage/pannus junction in rheumatoid arthritis etc.

Elastase is one of the proteases. It is an enzyme capable of hydrolyzing the connective tissue component elastin, a property not contained by the bulk of the proteases present in mammals. It acts on a protein's nonterminal bonds which are adjacent to an aliphatic amino acid. Neutrophil elastase is of particular interest because it has the broadest spectrum of activity against natural connective tissue substrates. In particular, the elastase of the granulocyte is important because, as described above, granulocytes participate in acute inflammation and in acute exacerbation of chronic forms of inflammation which characterize many clinically important inflammatory diseases.

Proteases may be inactivated by inhibitors which block the active site of the enzyme by binding tightly thereto. Naturally occurring protease inhibitors form part of the control or defense mechanisms that are crucial to the well-being of an organism. Without these control mechanisms, the proteases would destroy any protein within reach. The naturally occurring enzyme inhibitors have been shown to have appropriate configurations which allow them to bind tightly to the enzyme. This configuration is part of the reason that inhibitors bind to the enzyme so tightly (see Stroud, "A Family of Protein-Cutting Proteins" Sci. Am. July 1974, pp. 74-88). For example, one of the natural inhibitors, $\alpha_1$-Antitrypsin, is a glycoprotein contained in human serum that has a wide inhibitory spectrum covering, among other enzymes, elastase both from the pancreas and the PMN. This inhibitor is hydrolyzed by the proteases to form a stable acyl enzyme in which the active site is no longer available. Marked reduction in serum $\alpha_1$-antitrypsin, either genetic or due to oxidants, has been associated with pulmonary emphysema which is a disease characterized by a progressive loss of lung elasticity and resulting respiratory difficulty. It has been reported that this loss of lung elasticity is caused by the progressive, uncontrolled proteolysis or destruction of the structure of lung tissue by proteases such as elastase released from leukocytes. J. C. Powers, TIBS, 211 (1976).

Rheumatoid arthritis is characterized by a progressive destruction of articular cartilage both on the free surface bordering the joint space and at the erosion front built up by synovial tissue toward the cartilage. This destruction process, in turn, is attributed to the protein-cutting enzyme elastase which is a neutral protease present in human granulocytes. This conclusion has been supported by the following observations:

(1) Recent histochemical investigations showed the accumulation of granulocytes at the cartilage/pannus junction in rheumatoid arthritis; and

(2) a recent investigation of mechanical behavior of cartilage in response to attack by purified elastase demonstrated the direct participation of granulocyte enzymes, especially elastase, in rheumatoid cartilage destruction. H. Menninger et al., in Biological Functions of Proteinases, H. Holzer and H. Tschesche, eds. Springer-Verlag, Berlin, Heidelberg, New York, pp. 196-206, 1979.

Accordingly, an object of this invention is to discover new protease inhibitors, especially elastase inhibitors, useful for controlling tissue damage and various inflammatory or degenerative conditions mediated by proteases particularly elastase.

Another object of the present invention is to provide pharmaceutical compositions for administering the active substituted azetidinones as protease inhibitors especially human leukocyte elastase.

Still a further object of this invention is to provide a method of controlling inflammatory conditions by administering a sufficient amount of one or more of the active, substituted azetidinones in a mammalian species in need of such treatment.

## DETAILED DESCRIPTION OF THE INVENTION

This invention relates to potent elastase inhibitors of formula (I) which are useful in the prevention, control and treatment of inflammatory/degenerative conditions especially arthritis and emphysema.

A large number of the azetidinone derivatives of formula (I) are known antibiotics which have been described in patents and various publications.

The formula of the substituted azetidinones which are found to exhibit anti-inflammatory and antidegenerative activities by the present invention are represented as follows:

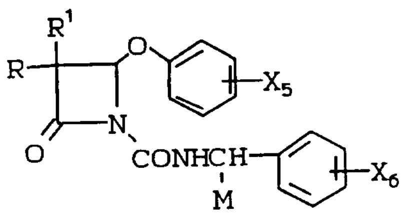

(I)

wherein:

R and $R^1$ independently are $C_{1-6}$ alkyl or $C_{1-6}$ alkoxy-$C_{1-6}$ alkyl;

M is

(1) hydrogen,
(2) $C_{1-6}$ alkyl,
(3) $C_{2-6}$ alkenyl, or
(4) $C_{1-6}$ alkoxy-$C_{1-6}$ alkyl;

$X_5$ is

(1) hydrogen,
(2) $C_{1-6}$ alkyl,
(3) halo-$C_{1-6}$ alkyl,
(4) $C_{2-6}$ alkenyl,
(5) $C_{2-6}$ alkynyl,
(6) carboxy,
(7) carboxy-$C_{1-6}$ alkyl,
(8) carboxy-$C_{1-6}$ alkylcarbonyl,
(9) carboxy-$C_{1-6}$ alkylcarbonylamino,
(10) carboxy-$C_{2-6}$ alkenyl,
(11) hydroxy-$C_{1-6}$ alkyl,
(12) $C_{1-6}$ alkylcarbonyl,
(13) $C_{1-6}$ alkylcarbonylamino, or
(14) di-($C_{1-6}$alkyl)amino-$C_{1-6}$alkyl; and

$X_5$ is

(1) hydrogen,
(2) $C_{1-6}$ alkyl,

(3) halo
(4) carboxy,
(5) $C_{1-6}$ alkoxy,
(6) phenyl,
(7) $C_{1-6}$ alkylcarbonyl,
(8) di-($C_{1-6}$alkyl)amino,
(9) phenoxy,
(10) methylenedioxy,
(11) 2,3-furanyl, or
(12) 2,3-thienyl; or

a pharmaceutically acceptable salt thereof.

Preferably, the compounds of the present invention are of formula (I) wherein;

R and $R^1$ independently are $C_{1-6}$ alkyl;

$X_5$ is carboxy or carboxy-$C_{1-6}$ alkyl.

Even more preferably, the compounds of the present invention are of formula (I) wherein R and $R^1$ independently are $C_{1-6}$ alkyl; M is $C_{1-3}$ alkyl or allyl; $X_5$ is carboxy or carboxy-$C_{1-6}$-alkyl and $X_5$ is hydrogen, $C_{1-6}$ alkyl, 3,4-methylenedioxy, or phenyl.

The most preferred compounds of the present invention are listed in the following table.

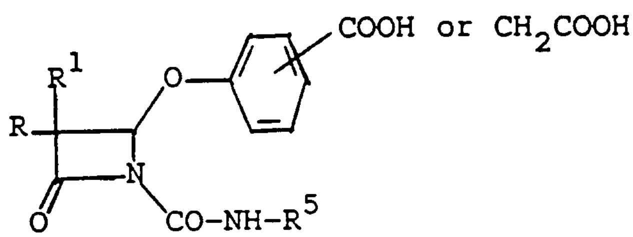

| R | $R^1$ | $R^5$ |
|---|---|---|
| $C_2H_5$ | $CH_3$ | $CH(CH_3)Ph$ |
| $C_2H_5$ | $C_2H_5$ | $CH_2$-(4-Ph-Ph) |
| $C_2H_5$ | $CH_3$ | $CH(C_2H_5)Ph$ |
| $C_2H_5$ | $C_2H_5$ | $CH(C_2H_5)Ph$ |
| $C_2H_5$ | $CH_2OCH_3$ | $CH(C_2H_5)Ph$ |
| $C_2H_5$ | $C_2H_5$ | $CH(C_3H_7)$-(4-$CH_3$-Ph) |
| $C_2H_5$ | $C_2H_5$ | $CH(C_3H_5)$-(4-$CH_3$-Ph) |
| $C_2H_5$ | $C_2H_5$ | $CH(C_3H_7)Ph$ |
| $C_2H_5$ | $C_2H_5$ | $CH(C_3H_7)$-(3,4-methylenedioxy-Ph) |
| $C_2H_5$ | $C_2H_5$ | $CH(C_3H_5)$-(3,4-methylenedioxy-Ph) |

In the above table, Ph represents phenyl or substituted phenyl as previously defined.

According to one aspect of the invention there is provided a process for the preparation of the compounds of Formula 1 which comprises

(1) reacting a compound of the following formula (B)

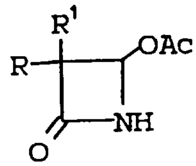

(B)

with a compound of the formula (C)

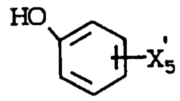

(C)

wherein

$X_5'$ is

(1) hydrogen
(2) $C_{1-6}$ alkyl,
(3) halo-$C_{1-6}$ alkyl,
(4) $C_{2-6}$ alkenyl,
(5) $C_{2-6}$ alkynyl,
(6) $C_{1-6}$ alkoxycarbonyl,
(7) $C_{1-6}$ alkoxycarbonyl-$C_{1-6}$ alkyl,
(8) $C_{1-6}$ alkoxycarbonyl-$C_{1-6}$ alkylcarbonyl,
(9) $C_{1-6}$ alkoxcarbonyl-$C_{1-6}$ alkylcarbonylamino,
(10) $C_{1-6}$ alkoxycarbonyl-$C_{2-6}$ alkenyl,
(11) hydroxyalkyl,
(12) $C_{1-6}$ alkylcarbonyl,
(13) $C_{1-6}$ alkylcarbonylamino, or
(14) di-($C_{1-6}$ alkyl)amino-$C_{1-6}$ alkyl

under basic conditions to afford a compound of the formula (D)

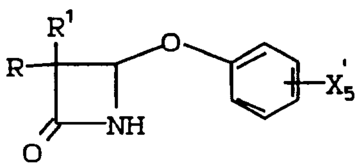

(D)

and (2) reacting compound (D) with a compound of the formula (E)

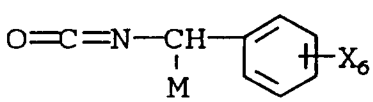

(E)

under basic conditions, and optionally converting $X_5'$ into $X_5$, to yield the compound of the formula (A)

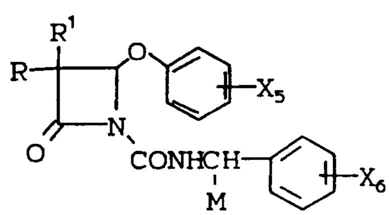

(A).

In this process step (1) is preferably carried out in the presence of an alkali metal hydroxide; step (2) is preferably carried out in the presence of a tri($C_{1-6}$alkyl)-amine and the conversion of $X_{5'}$ into $X_5$ is preferably accomplished in the presence of a strong acid.

The compounds of the present invention are either known or are prepared among other methods by the following representative schemes.

Scheme (a)

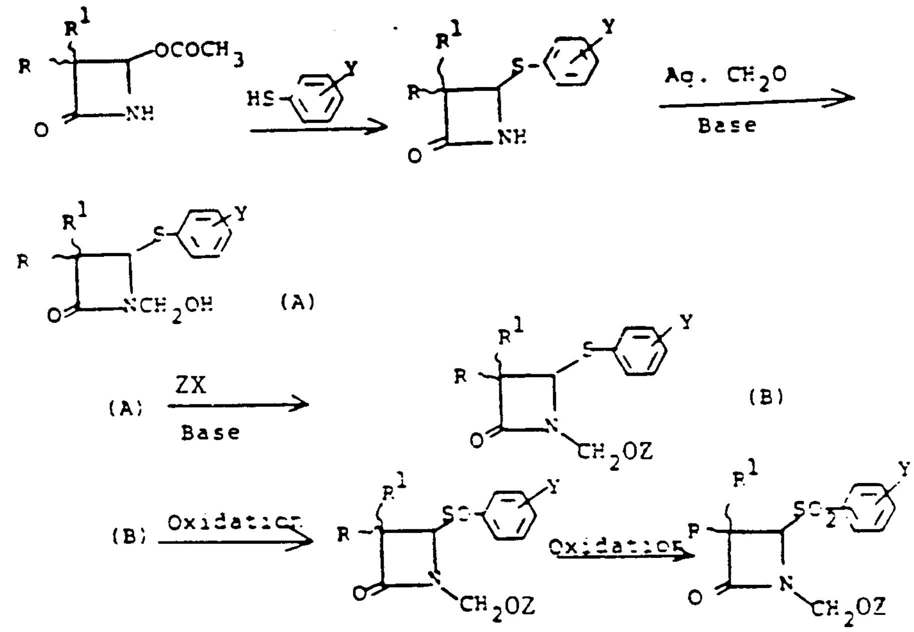


wherein

Y is $-NO_2$, $-CH_3$, $-OCH_3$, -Cl, -F, etc;

X is halo, e.g., Cl, Br or I;

Z is BCO or $BSO_2$.

Scheme (b)

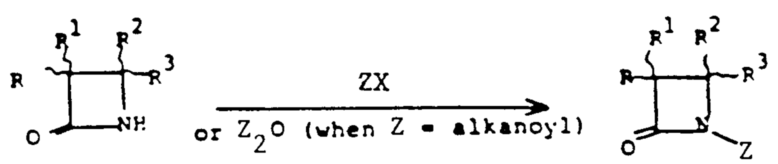


wherein

X is halo;

Z is as previously defined, e.g.,

$-SO_2$-(p-$NO_2$-Ph), $-COCH_3$, $-CH_2OTs$,

etc. wherein Ph represents phenyl or substituted phenyl.

Scheme (c)

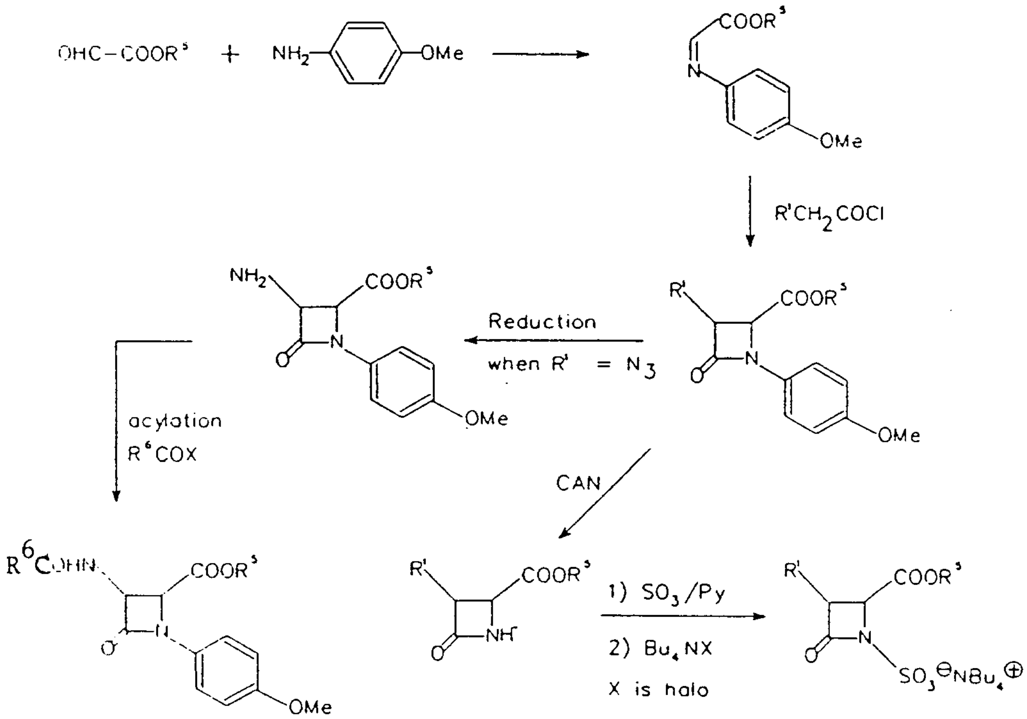


wherein
$R^6$ is H, $CF_3$, $CH_3$, etc.;
$R^5$ and $R^1$ are as previously defined; and
CAN is cerric ammonium nitrate.

Scheme (d)

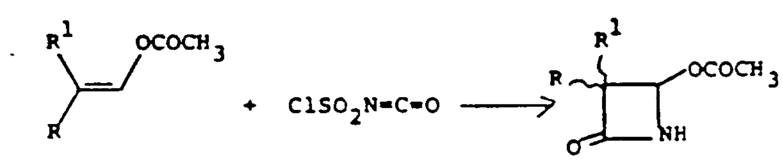

Scheme (e) as taught by M. A. Krook and M. J. Miller (J. Org. Chem., 1985, 50, 1126-1128), the following type of compounds can be prepared.

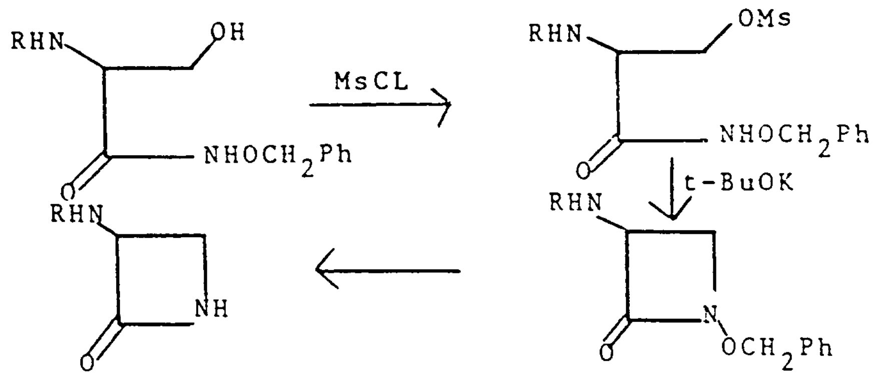


Scheme (f) as taught by Hart, D. J. et al., (J. Org. Chem., 48, pp. 289-294, 1983); the following class of compounds can be prepared.

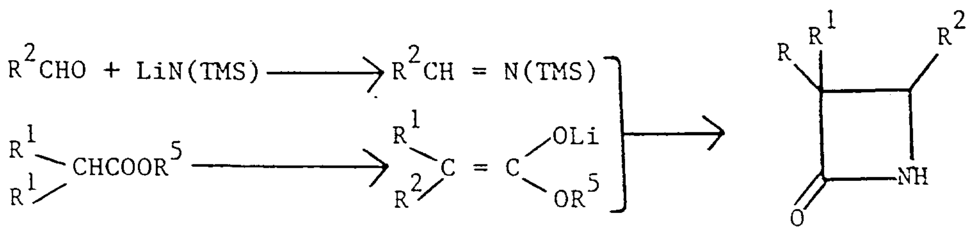

wherein $R^5$ is as previously defined; and TMS is trimethylsilyl.

Scheme (g) as taught by P. J. Reider and E. J. J. Grabowski (Tet. Lett., 23, p. 2293, 1982); the following groups of compounds can be prepared.

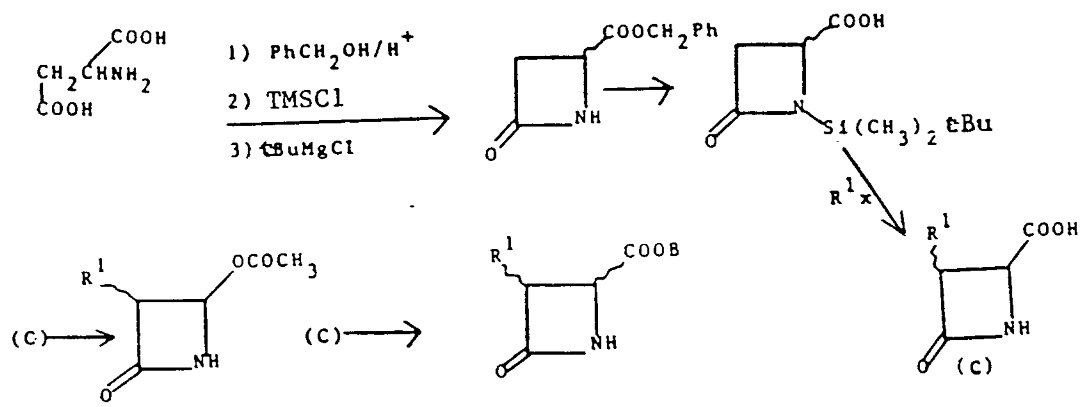


wherein $R^1$ is as previously defined.

Scheme (h) as illustrated by Examples 1 and 2 :

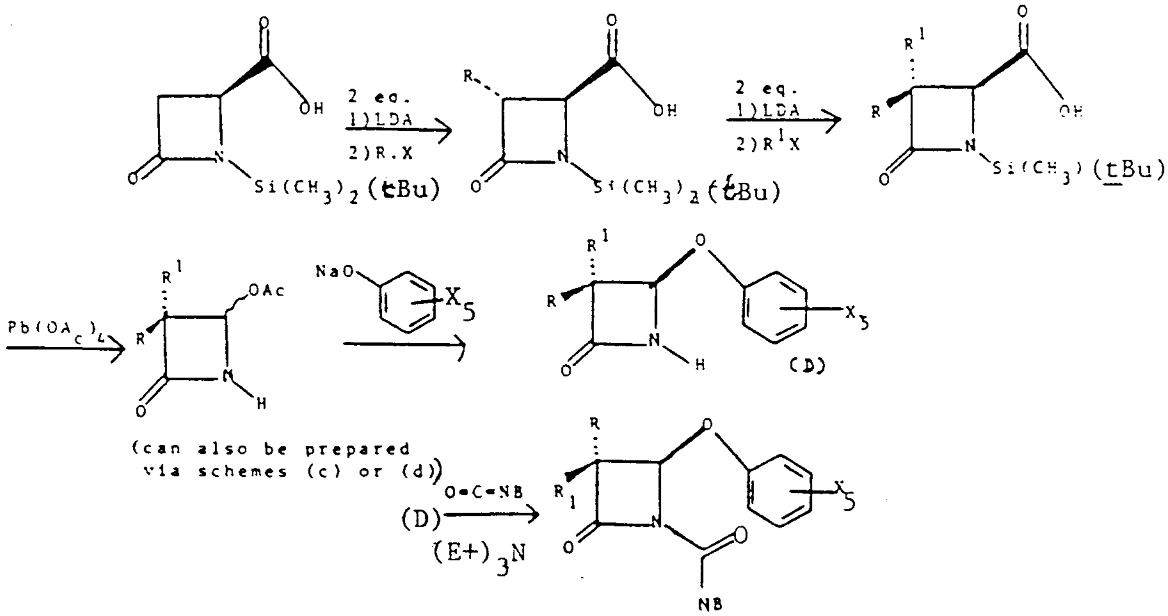


This invention also relates to a method of treating inflammation in patients using a compound of Formula (I), particularly a preferred compound as the active constituent.

It has been found that the compounds of Formula (I) are effective inhibitors of the proteolytic function of human granulocyte elastase as shown below:

Table II

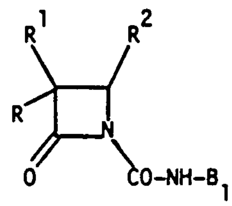

| R | $R^1$ | $R^2$ | $B_1$ | $k_{obs}/I$ |
|---|---|---|---|---|
| $C_3H_7$ | $CH_3$ | O-(4-COOH-Ph) | $CH_2Ph$ | 1900 |
| $C_2H_5$ | $CH_3$ | O-(4-COOH-Ph) | $CH(CH_3)Ph$ | 15,000 |
| $C_2H_5$ | $C_2H_5$ | $O-(4-CO(CH_2)_2COOH-Ph)$ | $CH_2(4-Ph-Ph)$ | 107,045 |
| $C_2H_5$ | $C_2H_5$ | O-(4-COOH-Ph) | $CH_2(4-Ph-Ph)$ | 37,000 |
| $C_2H_5$ | $CH_2OCH_3$ | O-(4-COOH-Ph) | $CH_2(4-Ph-Ph)$ | 44,533 |
| $C_2H_5$ | $C_2H_5$ | $O-(2-CH_2OH-Ph)$ | $CH_2Ph$ | 2961 |
| $C_2H_5$ | $C_2H_5$ | $O-(4-CH_2COOH-Ph)$ | $CH_2Ph$ | 3175 |
| $C_2H_5$ | $C_2H_5$ | $O-(4-NHCOCH_3-Ph)$ | $CH_2Ph$ | 3503 |
| $C_2H_5$ | $C_2H_5$ | $O-(4-NHCOCH_2CH_2COOH-Ph)$ | $CH_2Ph$ | 2568 |
| $C_2H_5$ | $C_2H_5$ | $O-(4-CH_3CO-Ph)$ | $CH_2-(4-COOH-Ph)$ | 2807 |
| $C_2H_5$ | $C_2H_5$ | $O-(4-CH_3CO-Ph)$ | $CH_2(4-CH_3CO-Ph)$ | 5916 |

$ID_{50}$ is the effective dosage in micrograms per milliliter (μg/ml) for 50% inhibition of the enzyme activity two minutes after time zero. Ki is the concentration of the inhibitor (micromolar, μM) giving 50% of the control enzyme activity. $k_{obs}/I$ ($M^{-1}$ $sec^{-1}$) is the second order rate constant of inactivation of the enzyme.

| R | $R^1$ | $R^2$ | $B_1$ | $k_{obs}/I$ |
|---|---|---|---|---|
| $C_2H_5$ | $C_2H_5$ | O-Ph | $CH_2$-(4-COOH-Ph) | 1501 |
| $C_2H_5$ | $CH_3$ | O-(4-COOH-Ph) | $CH_2Ph$ | 2000 |
| $C_3H_7$ | $C_2H_5$ | O-(4-COOH-Ph) | $CH_2Ph$ | 3280 |
| $C_2H_5$ | $CH_2OCH_3$ | O-(4-COOH-Ph) | $CH_2Ph$ | 1900 |
| $C_2H_5$ | $C_2H_5$ | O-(4-COOH-Ph) | $CH_2$-(4-Cl-Ph) | 3419 |
| $C_2H_5$ | $C_2H_5$ | O-(4-COOH-Ph) | $CH_2$(4-$CH_3$-Ph) | 3965 |
| $C_2H_5$ | $C_2H_5$ | O-(4-COOH-Ph) | $CH_2$(4-F-Ph) | 2337 |
| $C_2H_5$ | $C_2H_5$ | O-(4-COOH-Ph) | $CH_2$(4-$OCH_3$-Ph) | 5162 |
| $C_2H_5$ | $CH_2OCH_3$ | O-(4-COOH-Ph) | $CH_2$-(3,4-methylene-dioxy-Ph) | 16,984 |

| R | $R^1$ | $R^2$ | $B_1$ | $k_{obs}/I$ |
|---|---|---|---|---|
| $C_2H_5$ | $C_2H_5$ | O-(3-COOH-Ph) | $CH_2Ph$ | 1763 |
| $C_2H_5$ | $C_2H_5$ | O-(4-COOH-Ph) | $CH_2$-(4-PhO-Ph) | 12,036 |

Table II

(continued)

| R | $R^1$ | $R^2$ | $B_1$ | $k_{obs}/I$ |
|---|---|---|---|---|
| $C_3H_7$ | $C_3H_7$ | O-(4-COOH-Ph) | $CH_2Ph$ | 2,500 |
| $C_2H_5$ | $CH_3$ | O-(4-COOH-Ph) | CH(Et)-5-benzofuryl | 750,000 |
| $C_2H_5$ | $CH_2OCH_3$ | O-(4-COOH-Ph) | CH(nPr)Ph | 75,000 |
| $C_2H_5$ | $C_3H_7$ | O-(4-CO$(CH_2)_2$COOH-Ph) | CH(Et)Ph | 87,000 |
| $C_2H_5$ | $C_3H_7$ | O-(4-$CH_2$COOH-Ph) | CH(Et)Ph | 54,000 |

Me represents $CH_3$

Ph represents phenyl

Pr represents propyl

Bu represents butyl

Table III

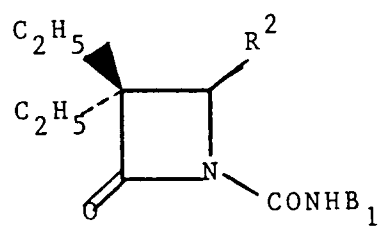

Table III

(Continued)

| $R^2$ | $B_1$ | $k_{obs}/I$ |
|---|---|---|
| O-(4-COOH-Ph) | $CH_2Ph(4\text{-}tBu)$ | 21,774 |
| O-(4-COOH-Ph) | $CH_2Ph(3\text{-}CH_3)$ | 6932 |
| O-(4-COOH-Ph) | $CH_2Ph(4\text{-}i\text{-}Pr)$ | 18,846 |
| O-(4-COOH-Ph) | $CH_2Ph(4\text{-}COMe)$ | 5916 |
| $O\text{-}(2\text{-}(CH_2)_3NMe_2\text{-}Ph)$ | $CH_2Ph$ | 629 |
| $O\text{-}(4\text{-}CH_2COOH\text{-}Ph)$ | $CH_2Ph(4\text{-}Ph)$ | 28,870 |

Table IV

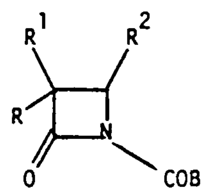

| R | $R^1$ | $R^2$ | B | $k_{obs}/I$ |
|---|---|---|---|---|
| $C_3H_7$ | $C_3H_7$ | O-(4-COOH-Ph) | 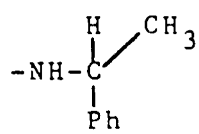 | 1446 (lower ref.)<br>4324 (higher ref.) |

Table V

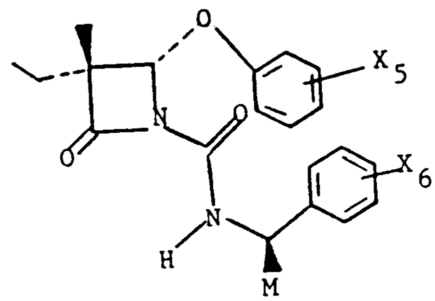

| $X_5$ | M | $X_6$ | $k_{obs}/I$ |
|---|---|---|---|
| 4-COOH | Et | H | 92,000 |
| 4-COOH | nPr | H | 152,000 |
| 4-COOH | $CH_2OMe$ | H | 6,094 |
| 4-$CH_2COOH$ | Et | H | 140,000 |
| 4-COOH | Me | 4-Me | 47,000 |
| 4-COOH | Et | 4-Me | -- |
| 4-$CH_2COOH$ | nPr | H | 227,000 |
| 4-COOH | nPr | $CH_3$ | -- |
| 4-COOH | nPr | H | 120,000 |
| 4-COOH | Et | 3,4-($OCH_2O$) | -- |
| 4-$CH_2COOH$ | nBu | H | -- |
| 4-COOH | allyl | H | -- |

Table VI

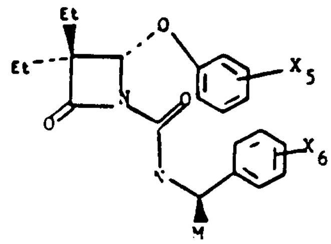

| $X_5$ | M | $X_6$ | $k_{obs}/I$ |
|---|---|---|---|
| 4-COOH | Me | H | 4016 |
| 4-COOH | Me | 4-Ph | 74,000 |
| 4-$CH_2$COOH | Me | H | 8,373 |
| 4-COOH | Me(s) | 4-Ph | 49246 |
| 4-COOH | Et | 4-Ph | 26382 |
| 4-COOH | Et | H | 76204 |
| 4-CO-$(CH_2)_2$-COOH | Me | H | 37084 |
| 4-CO-$(CH_2)_2$COOH | Et | H | 272190 |
| 4-COOH | nPr | H | 116060 |
| 4-$CH_2$COOH | Et | H | 126,000 |
| 3-$CH_2$COOH | Me | H | 5885 |
| 4-CH=CH-COOH | Me | H | 9101 |
| 4-COOH | $CH_2$OMe(S) | H | 6981 |
| 4-$CH_2$COOH | $CH_2$OMe(S) | H | |
| 4-COOH | Me | -Me | 10680 |
| 4-COOH | iPr(S) | H | 4743 |
| 4-COOH | iPr | H | 177075 |
| 4-$CH_2$COOH | nPr | H | 188,000 |
| 4-$CH_2$COOH | $CH_2$OMe(R) | H | 11004 |

Table VI

(Continued)

| $X_5$ | M | $X_6$ | $k_{obs}/I$ |
|---|---|---|---|
| 3-$CH_2COOH$ | Et | 4-Me | |
| 4-$(CH_2)_2COOH$ | Me | H | 9481 |
| 3-$CH_2COOH$ | Et | H | 81018 |
| 4-COOH | $CH_2OMe$(R) | H | 6981 |
| 4-COOH | Et | 3-Me | |
| 4-$CH_2COOH$ | Et | 3-Me | |
| 4-$CO(CH_2)_2COOH$ | allyl | 4-Me | |
| 4-COOH | Me | 4-Me | |
| 4-$CH_2COOH$ | Et | 3-Cl | |
| 4-COOH | Et | 3-Cl | |
| 4-COOH | allyl | 3-Me | |
| 4-COOH | nPr | 3-Me | |
| 4-$CH_2COOH$ | allyl | 4-Me | 664,000 |
| 3-$CH_2COOH$ | allyl | 4-Me | |
| 4-$CH_2COOH$ | allyl | 3-Me | |
| 4-$CH_2COOH$ | nPr | 3-Me | |
| 4-$CO(CH_2)_2COOH$ | nPr | 4-Me | |
| 3-$CH_2COOH$ | allyl | H | |
| 3-$CH_2COOH$ | $CH_2OMe$(S) | H | |
| 4-COOH | allyl | H | |
| 4-$CH_2COOH$ | allyl | H | |
| 4-COOH | Et | 4-Me | |
| 4-COOH | Et(S) | 4-Me | |
| 4-COOH | allyl | 4-Me | |
| 4-COOH | nPr | 4-Me | 389,000 |
| 3-$CH_2COOH$ | nPr | 4-Me | |
| 4-$CH_2COOH$ | nPr | 4-Me | 557,000 |

Table VI

(Continued)

| $X_5$ | M | $X_6$ | $k_{obs}/I$ |
|---|---|---|---|
| $3-CH_2COOH$ | Et | 4-Cl | |
| 4-COOH | Et | 4-Cl | |
| $4-CH_2COOH$ | Et | 4-Me | |
| $3-CH_2COOH$ | Et | 3-Cl | |
| 4-COOH | allyl | 3,4-methylenedioxy | |
| 4-COOH | nPr | 3,4-methylenedioxy | |
| $4-CH_2COOH$ | allyl | 3,4-methylenedioxy | 605,000 |
| $4-CH_2COOH$ | nPr | 3,4-methylenedioxy | 867,000 |
| $3-CH_2COOH$ | nPr | H | |
| 4-COOH | Et | 3,4-methylenedioxy | |
| $4-CH_2COOH$ | Et | 3,4-methylenedioxy | |
| 4-COOH | nBu | H | |

Protocol - Enzyme Assays for the Inhibition of Human Polymorphonuclear Leukocyte Elastase Via Hydrolysis of N-t-Boc-alanyl-alanyl-prolylalanine-p-nitroanilide (Boc-AAPAN) or N-t-Boc-alanyl-prolylvaline-p-nitroanilide (Boc-AAPVN) Reagent:

0.05M TES (N-tris[hydroxymethyl]methyl-2-amino-ethanesulfonic acid) Buffer, pH 7.5.

0.2 mM Boc-AAPAN or Boc-AAPVN.

To prepare substrate, the solid was first dissolved in 10.0 ml DMSO. Buffer at pH 7.5 was then added to a final volume of 100 ml.

Crude extract of human polymorphonuclear leukocytes (PMN) containing elastase activity.

Inhibitors (azetidinones) to be tested dissolved in DMSO just before use.

Assay Procedure:

To 1.0 ml of 0.2 mM Boc-AAPAN in a cuvette, 0.01-0.1 ml of DMSO with or without inhibitor was added. After mixing, a measurement was taken at 410 mμ to detect any spontaneous hydrolysis due to presence of test compound. 0.05 Milliliters of PMN extract was then added and the ΔOD/min at 410 mμ was measured and recorded. Beckman model 35 spectrophotometer was used.

Results:

Results in Table I were reported as $ID_{50}$, effective dosage in micrograms per milliliter (μg/ml) for 50% inhibition of the enzyme activity 2 minutes after zero time.

Results were also expressed as Ki, the micromolar concentration of the inhibitor (μM) giving 50% of the control enzyme activity; or as $^{k}obs/I$ which is the second order rate constant in per mole per second for inactivation of the enzyme.

Comments:

The elastase activity in the crude PMN extract may vary from one preparation to another. A control of each new batch is run, and the volume added in the assay procedure is adjusted according to activity.

Accordingly, the compounds of Formula (I) can be used to reduce inflammation and relieve pain in diseases such as emphysema, rheumatoid arthritis, osteoarthritis, gout, bronchial inflammation, atherosclerosis, sepsis, septicemia, shock, periodontitis, cystic fibrosis, infectious arthritis, rheumatic fever and the like.

According to a further aspect of the invention there is provided a pharmaceutical composition for the inhibition of human leukocyte elastase comprising a nontoxic therapeutically effective amount of a compound of formula (I) together with a pharmaceutically acceptable carrier.

For treatment of inflammation, fever or pain, the compounds of Formula (I) may be administered orally, topically, parenterally, by inhalation spray or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, dogs, cats, etc., the compounds of the invention are effective in the treatment of humans.

The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparation. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadeca-ethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyoxyethylene sorbitan monooleate. The said aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspension may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oils, or a mineral oil, for example liquid paraffin or mixtures of these. Suitable emulsifying agents may be naturally-occurring gums, for

example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds of Formula (I) may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc., containing the anti-inflammatory agents are employed.

The amount of active ingredient that may be combined with the carrier materials to produce a single dosage form will vary depending upon the host treated and the particular mode of administration. For example, a formulation intended for the oral administration of humans may contain from 5 mg to 5 gm of active agent compounded with an appropriate and convenient amount of carrier material which may vary from about 5 to about 95 percent of the total composition. Dosage unit forms will generally contain between from about 25 mg to about 500 mg of active ingredient.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

## EXAMPLE 1

### (3R,4S)-1-(benzylaminocarbonyl)-3-ethyl-3-methyl-4-(4-carboxy)phenoxyazetidin-2-one

Step A: Preparation of (3R,4S)-1-t-butyldimethylsilyl-3-methylazetidin-2-one-4-carboxylic acid

To a solution of 27.5 ml of diisopropylamine in 150 ml of THF at -20°C was added 73.5 ml of 2.4N n-butyl lithium in hexane. After 15 minutes, the solution was cooled to -70°C and a solution of 20 gm of (4S)-1-t-butyldimethylsilylazetidin-2-one-4-carboxylic acid in 75 mL of THF was added. The solution was warmed to -20°C for 15 minutes before a solution of 13.5 mL of methyl iodide in 20 mL of THF was added. After 30 minutes at -20 to 0°C, the reaction was diluted with 300 mL of ether and then poured into a mixture of ice and 400 mL of 1N HCl. The layers were separated and the aqueous layer extracted with ether. The ether layers were washed with brine, dried over sodium sulfate and evaporated. The residue was crystallized from hexane to give 12-15 gms of (3R,4S)-1-t-butyldimethylsilyl-3-methylazetidin-2-one-4-carboxylic acid.
NMR ($CDCl_3$): δ .14 (2, 3H), .32 (s, 3H), .91 (d, 3H), .98 (s, 9H), 3.34 (dq, 1H), 3.71 (d, 1H)

Step B: Preparation of (3R,4S)-1-t-butyldimethylsilyl-3-ethyl-3-methylazetidin-2-one-4-carboxylic acid

To a solution of 13 mL of diisopropylamine in 75 mL of THF at -20°C was added 35 mL of 2.4 M n-butyl lithium in hexane. After 15 minutes the solution was cooled to -70°C and a solution of 10 gms of (3R,4S)-1-t-butyldimethylsilyl-3-methylazetidin-2-one-4-carboxylic acid in 50 mL of THF was added. The solution was warmed to -20°C for 15 minutes and a solution of 6.7 mL of ethyl iodide in 10 mL of THF was added. After 30 minutes at -20° to 0°C the reaction was diluted with ether and poured into a mixture of ice and 1 N HCl. The layers were separated and the aqueous layer extracted with ether. The ether layers were each washed with brine, dried over sodium sulfate and evaporated. The residue was crystallized from a minimum amount of hexane to give 8.8 gms of (3R,4S)-1-t-butyldimethylsilyl-3-ethyl-3-methylazetidin-2-one-4-carboxylic acid.
NMR($CDCl_3$): δ .15 (s, 3H), .31 (s, 3H), .98 (s, 9H), 1.04 (t, 3H), 1.22 (s, 3H), 1.78 (q, 2H), 3.94 (s, 1H).

Step C: Preparation of (3R, 4S)-3-ethyl-3-methyl-4-(4-carbo-t-butoxy)phenoxyazetidin-2-one

To a solution of 13.0 gms of (3R, 4S)-1-t-butyldimethylsilyl-3-ethyl-3-methylazetidin-2-one-4-carboxylic acid in 75 mL of DMF and 15 mL of acetic acid under $N_2$ was added 23 gms of lead tetraacetate. The reaction was heated at 45-50 °C for 18 hours and then poured into ice water and extracted into 2 portions of ether. The ether layers were washed with water, dilute sodium bicarbonate solution and brine, dried over sodium sulfate and evaporated to give 13 gm of crude oil containing a mixture of (3R, 4S) and (3R, 4R)-4-acetoxy-3-ethyl-3-methylazetidin-2-one. To this mixture in 50 mL of acetone was slowly added a solution of 14 gms of t-butyl 4-hydroxybenzoate in 50 mL of acetone, 5 mL of water and 29 mL of 2N sodium hydroxide. The reaction was stirred at room temperature for 64 hours and then diluted with water and extracted with 2 portions of ether. The ether layers were washed with brine, dried over sodium sulfate and evaporated. The residue was prep LC'ed with 15-25% ethylacetate/hexanes to give 6.3 gm of the higher $R_f$ (4R) ether and 1.5 gm of the desired (3R, 4S)-3-ethyl-3-methyl-4-(4-carbo-t-butoxy)phenoxyazetidin-2-one.
NMR ($CDCl_3$): δ 1.0 (t, 3H), 1.38 (s, 3H), 1.54 (s, 9H), 1.6-2.0 (m, 2H), 5.30 (s, 1H) 6.7 (brs, 1H), 6.78 (d, 2H), 7.90 (d, 2H).

Step D: Preparation of (3R, 4S)-1-(benzylaminocarbonyl)-3-ethyl-3-methyl-4-(4-carbo-t-butoxy)-phenoxyazetidin-2-one

To a solution of 1.5 gm of (3R, 4S)-3-ethyl-3-methyl-4-(4-carbo-t-butoxy)phenoxyazetidin-2-one in 25 mL of methylene chloride was added 1.2 mL of benzyl isocyanate, 1.4 mL of triethylamine and 10 mg of 4-dimethylaminopyridine. The reaction was stirred at room temperature for 16 hours and then evaporated. The residue was flash chromatographed eluting with 10 to 25% EtoAc/Hexane to give 2.3 gm of (3R, 4S)-1-(benzylaminocarbonyl)-3-ethyl-3-methyl-4-(4-carbo-t-butoxy)phenoxy azetidin-2-one.
NMR ($CDCl_3$): δ .98 (t, 3H), 1.36 (s, 3H) 1.50 (s, 9H), 1.62 (m, 1H), 1.84 (m, 1H), 4.42 (d, 2H), 5.64 (s, 1H), 6.80 (brt, 1H), 7.06 (d, 2H), 7.24 (brs, 5H), 7.90 (d, 2H).

Step E: Preparation of (3R, 4S)-1-(benzylaminocarbonyl)-3-ethyl-3-methyl-4-(4-carboxy)phenoxyazetidin-2-one

To 2.3 gms of (3R, 4S)-1-(benzylaminocarbonyl)-3-ethyl-methyl-4-(4-carbo-t-butoxy) phenoxyazetidin-2-one in an ice bath under $N_2$ was added 5 mL of anisole and then 25 mL of precooled trifluoroacetic acid. After 1.5 hours at 0 °C, the volatiles were removed in vacuo without heating and the residue flash chromatographed using hexane, then 15% EtoAc/Hexane, then 1% HoAc in 15% EtoAc/hexanes to give after ether trituration 1.8 gms of (3R, 4S)-1-(benzylaminocarbonyl)-3-ethyl-3-methyl-4-(4-carboxy)-phenoxyazetidin-2-one
NMR ($CDCl_3$): δ 1.03 (t, 3H), 1.46 (s, 3H), 1.66 (m, 1H), 1.94 (m, 1H), 4.50 (d, 2H), 5.76 (s, 1H), 6.9 (brt, 1H), 7.05 (d, 2H), 7.25 (brs, 5H), 7.98 (d, 2H).

EXAMPLE 2

Starting with 3,3-diethyl-4-acetoxyazetidin-2-one as prepared in Scheme (d) followed by displacement of the acetate with the appropriate phenol and acylation of the nitrogen with the corresponding chiral isocyanate as shown in Scheme (h) and Example 1, steps C-E, the following compounds were prepared. The diastereomers obtained on acylation were separated by silica gel chromatography using 10-30% ethylacetate/hexane solvent mixtures.
(4S)-3,3-diethyl-1-((R)-α-ethylbenzylaminocarbonyl)-4-(4-carboxymethyl)phenoxyazetidin-2-one.

NMR ($CDCl_3$):δ 0.9 (t,3H,J = 7Hz), 0.94 (t,3H,J = 7Hz), 1.07 (t,3H,J = 7hz) 1.65 - 2.05 (m,6H), 3.58 (s,2H), 4.8 (q,1H, J = 8Hz), 5.58 (s,1H), 7.0 (d, 1H, J = 8Hz), 7.1 - 7.45 (m,9H)
(4S)-3,3-diethyl-1-((R)-α-n-propylbenzylaminocarbonyl)-4-(4-carboxymethyl)phenoxyazetidin-2-one.

NMR ($CDCl_3$): δ 0.91 (t,3H,J = 7Hz), 0.94 (t,3H,J = 7Hz), 1.07 (t,3H,J = 7hz) 1.34 (m,2H), 1.65-2.05 (m,6H), 3.57 (s,2H), 4.88 (q, 1H, J = 7Hz), 5.58 (s,1H), 7.0 (d, 1H, J = 7Hz) 7.1 - 7.5 (m, 9H)
(4S)-3,3-diethyl-1-((R)-α-allyl-(4-methyl)benzylaminocarbonyl)-4-(4-carboxymethyl)phenoxyazetidin-2-one.

NMR ($CDCl_3$): δ 0.96 (t,3H,J = 7Hz), 1.07 (t,3H,J = 7Hz), 1.7 - 2.1 (m, 4H), 2.32 (s, 3H), 2.57 (t,2H, J = 7Hz), 3.58 (s, 2H), 4.95 (q, 1H, J = 7Hz), 5.14 (m, 2H), 5.58 (s, 1H), 5.66 (m, 1H), 7.03 (d, 1H, J = 7Hz),

7.16 (s, 4H), 7.19 (s, 4H).

(4S)-3,3-diethyl-1-((R)-α-allyl(3,4-methylenedioxy)benzylaminocarbonyl)-4-(4-carboxymethyl)phenoxy-azetidin-2-one.

NMR ($CDCl_3$): δ 0.96 (t,3H,J=7Hz), 1.05 (t,3H,J=7Hz), 1.65 - 2.05 (m, 4H), 2.54 (t, 2H J=6Hz) 4.87 ((q, 1H, J=7Hz), 5.05 -5.2 (m, 2H), 5.58 (s, 1H), 5.66 (m, 1H), 5.94 (s, 2H), 6.76 (s, 3H), 6.98 (d, 1H, J=7Hz), 7.2 (m,4H)).

(4S)-3,3-diethyl-1-((R)-α-n-propyl(3,4-methylenedioxy)-benzylaminocarbonyl)-4-(4-carboxymethyl)phenoxy-azetidin-2-one.

NMR ($CDCl_3$): δ 0.9 (t,3H,J=7Hz), 0.94 (t,3H,J=7Hz), 1.06 (t, 3H J=7Hz), 1.3 (m, 2H), 1.65 - 2.1 (m, 6H), 3.58(s, 2H), 4.76(q, 1H, J=7hz), 5.58(s, 1H), 5.92 (s,2H), 6.15 (s, 3H) 6.88 (d, 1H, J=7Hz), 7.2 (m, 4H).

(4S)-3,3-dietnyl-1-((R)-α-n-propyl(4-methyl)benzylaminocarbonyl)-4-(4-carboxy)phenoxy-azetidin-2-one.

NMR ($CDCl_3$): δ 0.91 (t,3H,J=7Hz), 0.98 (t,3H,J=7Hz), 1.07 (t, 3H, J=7Hz) 1.32 (m, 2H), 1.65 - 2.1 (m, 6H), 2.33(s, 3H), 4.83(q, 1H, J=7hz), 5.71(s, 1H), 6.93 (d, 1H, J=7Hz), 7.16 (s, 4H), 7.25 (d,2H,J=8Hz), 8.04 (d, 2H, J=8Hz).

(4S)-3,3-diethyl-1-((R)-α-n-propyl(4-methyl)benzylaminocarbonyl)-4-(4-carboxymethyl)phenoxy-azetidin-2-one.

NMR ($CDCl_3$): δ 0.9 (t,3H,J=7Hz), 0.93 (t,3H,J=7Hz), 1.07 (t, 3H, J=7Hz) 1.28 (m, 2H), 1.7 - 2.1 (m, 6H), 2.33(s, 2H), 3.6 (s,2H), 4.81 (q, 1H, J=7hz), 5.56 (s, 1H), 6.93 (d, 1H, J=7Hz), 7.15 (s, 4H), 7.2 (s, 4H).

## Claims

**Claims for the following Contracting States : AT, BE, CH, DE, FR, GB, IT, LI, LU, NL, SE**

**1.** A compound of the formula (I)

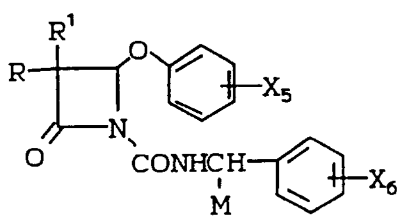

(I)

wherein:

R and $R^1$ independently are $C_{1-6}$ alkyl or $C_{1-6}$ alkoxy-$C_{1-6}$ alkyl;

M is

(1) hydrogen,
(2) $C_{1-6}$ alkyl,
(3) $C_{2-6}$ alkenyl, or
(4) $C_{1-6}$ alkoxy-$C_{1-6}$ alkyl;

$X_5$ is

(1) hydrogen,
(2) $C_{1-6}$ alkyl,
(3) halo-$C_{1-6}$ alkyl,
(4) $C_{2-6}$ alkenyl,
(5) $C_{2-6}$ alkynyl,
(6) carboxy,
(7) carboxy-$C_{1-6}$ alkyl,
(8) carboxy-$C_{1-6}$ alkylcarbonyl,
(9) carboxy-$C_{1-6}$ alkylcarbonylamino,
(10) carboxy-$C_{2-6}$ alkenyl,
(11) hydroxy-$C_{1-6}$ alkyl,
(12) $C_{1-6}$ alkylcarbonyl,
(13) $C_{1-6}$ alkylcarbonylamino, or
(14) di-($C_{1-6}$alkyl)amino-$C_{1-6}$alkyl; and

$X_5$ is

(1) hydrogen,
(2) $C_{1-6}$ alkyl,

(3) halo
(4) carboxy,
(5) $C_{1-6}$ alkoxy,
(6) phenyl,
(7) $C_{1-6}$ alkylcarbonyl,
(8) di-($C_{1-6}$alkyl)amino,
(9) phenoxy,
(10) methylenedioxy,
(11) 2,3-furanyl, or
(12) 2,3-thienyl; or

a pharmaceutically acceptable salt thereof.

**2.** A compound of Claim 1 wherein:
R and $R^1$ independently are $C_{1-6}$ alkyl;
and
$X_5$ is carboxy or carboxy-$C_{1-6}$ alkyl.

**3.** A compound of Claim 2 wherein:
M is $C_{1-3}$ alkyl or allyl; and
$X_5$ is hydrogen, $C_{1-6}$ alkyl,
3,4-methylenedioxy, or phenyl.

**4.** A compound of Claim 3 wherein:
R is ethyl; and
$R^1$ is methyl or ethyl.

**5.** A compound of Claim 4 wherein:
R and $R^1$ are ethyl;
M is n-propyl;
$X_5$ is 4-carboxymethyl; and
$X_5$ is 4-methyl, or 3,4-methylenedioxy.

**6.** A pharmaceutical composition for the inhibition of human leukocyte elastase which comprises a nontoxic therapeutically effective amount of a compound of Claim 1 and a pharmaceutically acceptable carrier.

**7.** A composition of Claim 6 wherein:
R is ethyl;
$R^1$ is methyl or ethyl;
M is $C_{1-3}$ alkyl or allyl;
$X_5$ is carboxy or carboxy-$C_{1-6}$ alkyl;
and
$X_5$ is hydrogen $C_{1-6}$ alkyl,
3,4-methylenedioxy or phenyl.

**8.** A composition of Claim 7 wherein:
$R^1$ is ethyl;
M is n-propyl;
$X_5$ is 4-carboxymethyl; and
$X_5$ is 4-methyl, or 3,4-methylenedioxy.

**9.** A process for the preparation of the compounds of Claim 1 which comprises

(1) reacting a compound of the following formula (B)

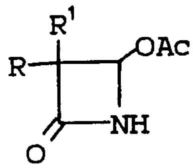

(B)

with a compound of the formula (C)

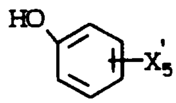

(C)

wherein

$X_5$' is

(1) hydrogen
(2) $C_{1-6}$ alkyl,
(3) halo-$C_{1-6}$ alkyl,
(4) $C_{2-6}$ alkenyl,
(5) $C_{2-6}$ alkynyl,
(6) $C_{1-6}$ alkoxycarbonyl,
(7) $C_{1-6}$ alkoxycarbonyl-$C_{1-6}$ alkyl,
(8) $C_{1-6}$ alkoxycarbonyl-$C_{1-6}$ alkylcarbonyl,
(9) $C_{1-6}$ alkoxycarbonyl-$C_{1-6}$ alkylcarbonylamino,
(10) $C_{1-6}$ alkoxycarbonyl-$C_{2-6}$ alkenyl,
(11) hydroxyalkyl,
(12) $C_{1-6}$ alkylcarbonyl,
(13) $C_{1-6}$ alkylcarbonylamino, or
(14) di-($C_{1-6}$ alkyl)amino-$C_{1-6}$ alkyl

under basic conditions to afford a compound of the formula (D)

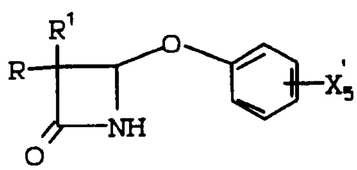

(D)

and (2) reacting compound (D) with a compound of the formula (E)

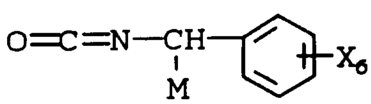

(E)

under basic conditions, and optionally converting $X_5$' into $X_5$, to yield the compound of the formula (A)

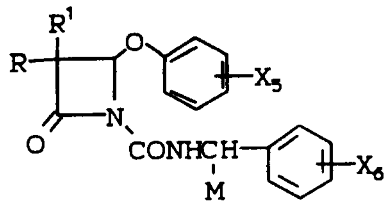

(A).

**10.** A process of Claim 9 wherein:

Step (1) is in the presence of an alkali metal hydroxide.

Ste (2) is in the presence of a tri($C_{1-6}$ alkyl)-amine; and

the conversion of $X_5$' into $X_5$ is accomplished in the presence of a strong acid.

**Claims for the following Contracting States : ES, GR**

**1.** A process for the preparation of a compound of the formula (I)

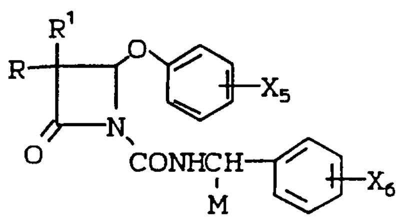

(I)

wherein:

R and $R^1$ independently are $C_{1-6}$ alkyl or $C_{1-6}$ alkoxy-$C_{1-6}$ alkyl;

M is

(1) hydrogen,
(2) $C_{1-6}$ alkyl,
(3) $C_{2-6}$ alkenyl, or
(4) $C_{1-6}$ alkoxy-$C_{1-6}$ alkyl;

$X_5$ is

(1) hydrogen,
(2) $C_{1-6}$ alkyl,
(3) halo-$C_{1-6}$ alkyl,
(4) $C_{2-6}$ alkenyl,
(5) $C_{2-6}$ alkynyl,
(6) carboxy,
(7) carboxy-$C_{1-6}$ alkyl,
(8) carboxy-$C_{1-6}$ alkylcarbonyl,
(9) carboxy-$C_{1-6}$ alkylcarbonylamino,
(10) carboxy-$C_{2-6}$ alkenyl,
(11) hydroxy-$C_{1-6}$ alkyl,
(12) $C_{1-6}$ alkylcarbonyl,
(13) $C_{1-6}$ alkylcarbonylamino, or
(14) di($C_{1-6}$alkyl)amino-$C_{1-6}$alkyl; and

$X_5$ is

(1) hydrogen,
(2) $C_{1-6}$ alkyl,
(3) halo
(4) carboxy,
(5) $C_{1-6}$ alkoxy,

(6) phenyl,
(7) $C_{1-6}$ alkylcarbonyl,
(8) di($C_{1-6}$alkyl)amino,
(9) phenoxy,
(10) methylenedioxy,
(11) 2,3-furanyl, or
(12) 2,3-thienyl; or

a pharmaceutically acceptable salt thereof which comprises

(1) reacting a compound of the following formula (B)

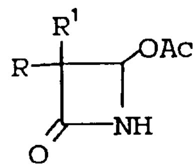

(B)

with a compound of the formula (C)

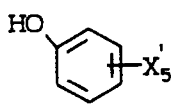

(C)

wherein

$X_5'$ is

(1) hydrogen
(2) $C_{1-6}$ alkyl,
(3) halo-$C_{1-6}$ alkyl,
(4) $C_{2-6}$ alkenyl,
(5) $C_{2-6}$ alkynyl,
(6) $C_{1-6}$ alkoxycarbonyl,
(7) $C_{1-6}$ alkoxycarbonyl-$C_{1-6}$ alkyl,
(8) $C_{1-6}$ alkoxycarbonyl-$C_{1-6}$ alkylcarbonyl,
(9) $C_{1-6}$ alkoxycarbonyl-$C_{1-6}$ alkylcarbonylamino,
(10) $C_{1-6}$ alkoxycarbonyl-$C_{2-6}$alkenyl,
(11) hydroxyalkyl,
(12) $C_{1-6}$ alkylcarbonyl,
(13) $C_{1-6}$ alkylcarbonylamino, or
(14) di-($C_{1-6}$ alkyl)amino-$C_{1-6}$ alkyl

under basic conditions to afford a compound of the formula (D)

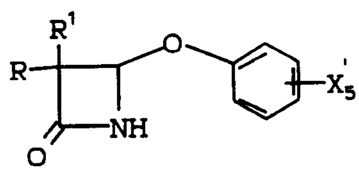

(D)

and (2) reacting compound (D) with a compound of the formula (E)

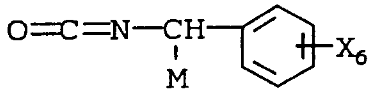

(E)

under basic conditions, and optionally converting $X_5$' into $X_5$, yield the compound of the formula (A)

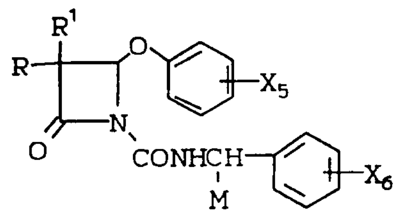

(A)

**2.** A process of Claim 1 wherein:
R and $R^1$ independently are $C_{1-6}$alkyl; and
$X_5$ is carboxy or carboxy-$C_{1-6}$alkyl.

**3.** A process of Claim 2 wherein:
M is $C_{1-3}$ alkyl or allyl; and
$X_5$ is hydrogen, $C_{1-6}$ alkyl, or 3,4-methylenedioxy or phenyl.

**4.** A process of Claim 3 wherein:
R is ethyl; and
$R^1$ is methyl or ethyl.

**5.** A process of Claim 4 wherein:
R and $R^1$ are ethyl;
M is n-propyl;
$X_5$ is 4-carboxymethyl; and
$X_5$ is 4-methyl, or 3,4-methylenedioxy.

**6.** A process of Claim 1 wherein:
Step (1) is in the presence of an alkali metal hydroxide;
Step (2) is in the presence of a tri($C_{1-6}$ alkyl)amine; and
the conversion of $X_5$' into $X_5$ is accomplished in the presence of a strong acid.

**Patentansprüche**
**Patentansprüche für folgende Vertragsstaaten : AT, BE, CH, DE, FR, GB, IT, LI, LU, NL, SE**

**1.** Eine Verbindung der Formel (I)

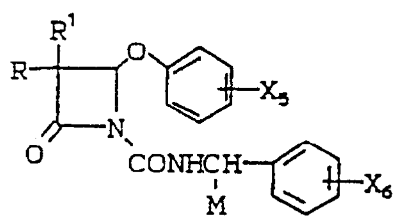

(I)

worin
R und $R^1$ unabhängig voneinander $C_{1-6}$ Alkyl oder $C_{1-6}$ Alkoxy-$C_{1-6}$ alkyl bedeuten;
M
(1) Wasserstoff,
(2) $C_{1-6}$ Alkyl,
(3) $C_{2-6}$ Alkenyl oder
(4) $C_{1-6}$ Alkoxy-$C_{1-6}$ alkyl bedeutet;
$X_5$

(1) Wasserstoff,
(2) $C_{1-6}$ Alkyl,
(3) Halogen-$C_{1-6}$ alkyl,
(4) $C_{2-6}$ Alkenyl,
(5) $C_{2-6}$ Alkinyl,
(6) Carboxy,
(7) Carboxy-$C_{1-6}$ alkyl,
(8) Carboxy-$C_{1-6}$ alkylcarbonyl,
(9) Carboxy-$C_{1-6}$ alkylcarbonylamino,
(10) Carboxy-$C_{2-6}$ alkenyl,
(11) Hydroxy-$C_{1-6}$ alkyl,
(12) $C_{1-6}$ Alkylcarbonyl,
(13) $C_{1-6}$ Alkylcarbonylamino, oder
(14) Di-($C_{1-6}$ alkyl)amino-$C_{1-6}$ alkyl bedeutet; und

$X_6$
(1) Wasserstoff,
(2) $C_{1-6}$ Alkyl,
(3) Halogen,
(4) Carboxy,
(5) $C_{1-6}$ Alkoxy,
(6) Phenyl,
(7) $C_{1-6}$ Alkylcarbonyl,
(8) Di-($C_{1-6}$ alkyl)amino,
(9) Phenoxy,
(10) Methylendioxy,
(11) 2,3-Furanyl, oder
(12) 2,3-Thienyl bedeutet; oder

ein pharmazeutisch annehmbares Salz hiervon.

**2.** Eine Verbindung des Anspruchs 1, worin
R und $R^1$ unabhängig voneinander $C_{1-6}$ Alkyl bedeuten; und
$X_6$ Carboxy oder Carboxy-$C_{1-6}$ alkyl bedeutet.

**3.** Eine Verbindung des Anspruchs 2, worin
M $C_{1-3}$ Alkyl oder Allyl, und
$X_6$ Wasserstoff, $C_{1-6}$ Alkyl, 3,4-Methylendioxy oder Phenyl bedeuten.

**4.** Eine Verbindung des Anspruchs 3, worin
R Ethyl und $R^1$ Methyl oder Ethyl bedeuten.

**5.** Eine Verbindung des Anspruchs 4, worin
R und $R^1$ Ethyl, M n-Propyl, $X_6$ 4-Carboxymethyl und
$X_6$ 4-Methyl oder 3,4-Methylendioxy bedeuten.

**6.** Eine pharmazeutische Zusammensetzung zur Hemmung der humanen Leukozyten-Elastase, welche eine untoxische, therapeutisch wirksame Menge einer Verbindung des Anspruchs 1 und eine pharmazeutisch annehmbare Trägersubstanz umfaßt.

**7.** Eine Zusammensetzung des Anspruchs 6, worin
R Ethyl, $R^1$ Methyl oder Ethyl, M $C_{1-3}$ Alkyl oder Allyl,
$X_6$ Carboxy oder Carboxy-$C_{1-6}$ alkyl und $X_6$ Wasserstoff, $C_{1-6}$ Alkyl, 3,4-Methylendioxy oder Phenyl bedeuten.

**8.** Eine Zusammensetzung des Anspruchs 7, worin
$R^1$ Ethyl, M n-Propyl, $X_6$ 4-Carboxymethyl und
$X_6$ 4-Methyl oder 3,4-Methylendioxy bedeuten.

**9.** Ein Verfahren zur Herstellung der Verbindungen des Anspruchs 1, umfassend

(1) Reagieren einer Verbindung der folgenden Formel (B)

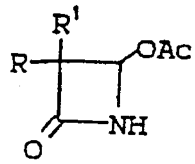

(B)

mit einer Verbindung der Formel (C)

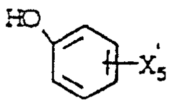

(C)

worin

$X_5'$

(1) Wasserstoff,
(2) $C_{1-6}$ Alkyl,
(3) Halogen-$C_{1-6}$ alkyl,
(4) $C_{2-6}$ Alkenyl,
(5) $C_{2-6}$ Alkinyl,
(6) $C_{1-6}$ Alkoxycarbonyl,
(7) $C_{1-6}$ Alkoxycarbonyl-$C_{1-6}$ alkyl,
(8) $C_{1-6}$ Alkoxycarbonyl-$C_{1-6}$ alkylcarbonyl,
(9) $C_{1-6}$ Alkoxycarbonyl-$C_{1-6}$ alkylcarbonylamino,
(10) $C_{1-6}$ Alkoxycarbonyl-$C_{2-6}$ alkenyl,
(11) Hydroxyalkyl,
(12) $C_{1-6}$ Alkylcarbonyl,
(13) $C_{1-6}$ Alkylcarbonylamino, oder
(14) Di-($C_{1-6}$ alkyl)amino-$C_{1-6}$ alkyl bedeutet;

unter basichen Bedingungen, um eine Verbindung der Formel (D)

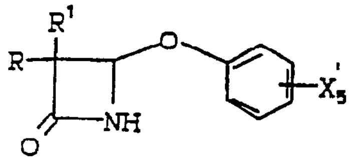

(D)

zu liefern, und

(2) Reagieren der Verbindung (D) mit einer Verbindung der Formel (E)

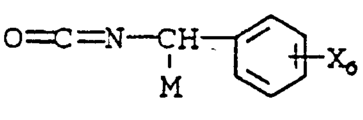

(E)

unter basischen Bedindungen, und gegebenenfalls Überführen von $X_5'$ in $X_5$, um die Verbindung der Formel (A) zu liefern

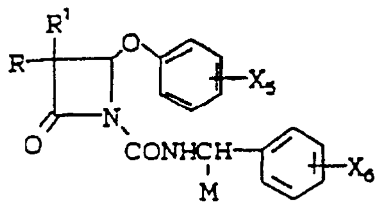

(A).

**10.** Ein Verfahren des Anspruchs 9, worin

die Stufe (1) in Gegenwart eines Alkalimetallhydroxids durchgeführt wird,
die Stufe (2) in Gegenwart eines Tri-($C_{1-6}$ alkyl)-amins durchgeführt wird, und
die Überführung von $X_5$' in $X_5$ in Gegenwart einer starken Säure ausgeführt wird.

**Patentansprüche für folgende Vertragsstaaten : ES, GR**

**1.** Ein Verfahren zur Herstellung einer Verbindung der Formel (I) worin

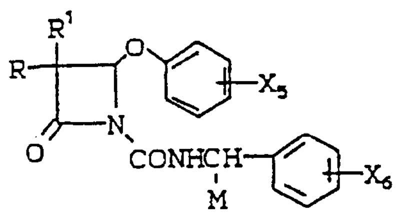

(I)

R und $R^1$ unabhängig voneinander $C_{1-6}$ Alkyl oder $C_{1-6}$ Alkoxy-$C_{1-6}$ alkyl bedeuten;

M

(1) Wasserstoff,
(2) $C_{1-6}$ Alkyl,
(3) $C_{2-6}$ Alkenyl oder
(4) $C_{1-6}$ Alkoxy-$C_{1-6}$ alkyl bedeutet;

$X_5$

(1) Wasserstoff,
(2) $C_{1-6}$ Alkyl,
(3) Halogen-$C_{1-6}$ alkyl,
(4) $C_{2-6}$ Alkenyl,
(5) $C_{2-6}$ Alkinyl,
(6) Carboxy,
(7) Carboxy-$C_{1-6}$ alkyl,
(8) Carboxy-$C_{1-6}$ alkylcarbonyl,
(9) Carboxy-$C_{1-6}$ alkylcarbonylamino,
(10) Carboxy-$C_{2-6}$ alkenyl,
(11) Hydroxy-$C_{1-6}$ alkyl,
(12) $C_{1-6}$ Alkylcarbonyl,
(13) $C_{1-6}$ Alkylcarbonylamino, oder
(14) Di-($C_{1-6}$ alkyl)amino-$C_{1-6}$ alkyl bedeutet; und

$X_5$

(1) Wasserstoff,
(2) $C_{1-6}$ Alkyl,
(3) Halogen,
(4) Carboxy,
(5) $C_{1-6}$ Alkoxy,
(6) Phenyl,
(7) $C_{1-6}$ Alkylcarbonyl,
(8) Di-($C_{1-6}$ Alkyl)amino,

(9) Phenoxy,
(10) Methylendioxy,
(11) 2,3-Furanyl, oder
(12) 2,3-Thienyl bedeutet; oder

eines pharmazeutisch annehmbaren Salzes hiervon, umfassend

(1) Reagieren einer Verbindung der folgenden Formel (B)

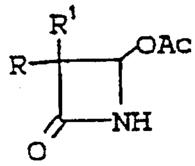

(B)

mit einer Verbindung der Formel (C)

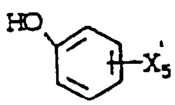

(C)

worin

$X_5$'

(1) Wasserstoff,
(2) $C_{1-6}$ Alkyl,
(3) Halogen-$C_{1-6}$ alkyl,
(4) $C_{2-6}$ Alkenyl,
(5) $C_{2-6}$ Alkinyl,
(6) $C_{1-6}$ Alkoxycarbonyl,
(7) $C_{1-6}$ Alkoxycarbonyl-$C_{1-6}$ alkyl,
(8) $C_{1-6}$ Alkoxycarbonyl-$C_{1-6}$ alkylcarbonyl,
(9) $C_{1-6}$ Alkoxycarbonyl-$C_{1-6}$ alkylcarbonylamino,
(10) $C_{1-6}$ Alkoxycarbonyl-$C_{2-6}$ alkenyl,
(11) Hydroxyalkyl,
(12) $C_{1-6}$ Alkylcarbonyl,
(13) $C_{1-6}$ Alkylcarbonylamino, oder
(14) Di-($C_{1-6}$ alkyl)amino-$C_{1-6}$ alkyl bedeutet;

unter basichen Bedingungen, um eine Verbindung der Formel (D)

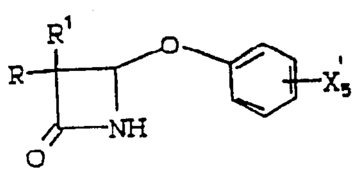

(D)

zu liefern, und

(2) Reagieren der Verbindung (D) mit einer Verbindung der Formel (E)

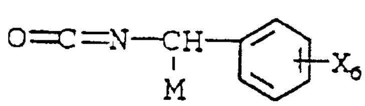

(E)

unter basischen Bedindungen, und gegebenenfalls Überführen von $X_5$' in $X_5$, um die Verbindung der Formel (A) zu liefern

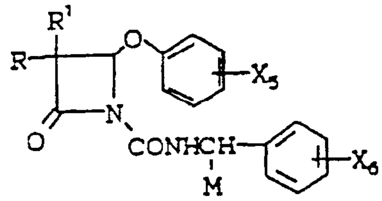

(A).

2. Ein Verfahren des Anspruchs 1, worin
R und $R^1$ unabhängig voneinander $C_{1-6}$ Alkyl bedeuten; und
$X_5$ Carboxy oder Carboxy-$C_{1-6}$ alkyl bedeutet.

3. Ein Verfahren des Anspruchs 2, worin
M $C_{1-3}$ Alkyl oder Allyl, und
$X_5$ Wasserstoff, $C_{1-6}$ Alkyl, 3,4-Methylendioxy oder Phenyl bedeuten.

4. Ein Verfahren des Anspruchs 3, worin
R Ethyl und $R^1$ Methyl oder Ethyl bedeuten.

5. Ein Verfahren des Anspruchs 4, worin
R und $R^1$ Ethyl, M n-Propyl, $X_5$ 4-Carboxymethyl und
$X_5$ 4-Methyl oder 3,4-Methylendioxy bedeuten.

6. Ein Verfahren des Anspruchs 1, worin
die Stufe (1) in Gegenwart eines Alkalimetallhydroxids durchgeführt wird,
die Stufe (2) in Gegenwart eines Tri-($C_{1-6}$ alkyl)-amins durchgeführt wird, und
die Überführung von $X_5$' in $X_5$ in Gegenwart einer starken Säure ausgeführt wird.

**Revendications**
**Revendications pour les Etats contractants suivants : AT, BE, CH, DE, FR, GB, IT, LI, LU, NL, SE**

1. Composé de formule (I)

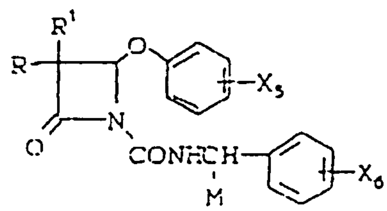

(I)

dans laquelle:
R et $R^1$ représentent indépendamment un alkyle en $C_1$ à $C_6$ ou un alcoxy en $C_1$ à $C_6$-alkyle en $C_1$ à $C_6$;
M représente
(1) un hydrogène,
(2) un alkyle en $C_1$ à $C_6$,
(3) un alcényle en $C_2$ à $C_6$ ou,
(4) un alcoxy en $C_1$ à $C_6$-alkyle en $C_1$ à $C_6$;
$X_5$ représente
(1) un hydrogène,
(2) un alkyle en $C_1$ à $C_6$,
(3) un halo-alkyle en $C_1$ à $C_6$,
(4) un alcényle en $C_2$ à $C_6$,
(5) un alcynyle en $C_2$ à $C_6$,

(6) un carboxy,
(7) un carboxy-alkyle en $C_1$ à $C_6$,
(8) un carboxy-alkyle en $C_1$ à $C_6$-carbonyle,
(9) un carboxy-alkyle en $C_1$ à $C_6$-carbonylamino,
(10) un carboxy-alcényle en $C_2$ à $C_6$,
(11) un hydroxy-alkyle en $C_1$ à $C_6$,
(12) un alkyle en $C_1$ à $C_6$-carbonyle,
(13) un alkyle en $C_1$ à $C_6$-carbonylamino, ou
(14) un di-(alkyle en $C_1$ à $C_6$) amino-alkyle en $C_1$ à $C_6$; et

$X_6$ représente
(1) un hydrogène,
(2) un alkyle en $C_1$ à $C_6$,
(3) un halo,
(4) un carboxy,
(5) un alcoxy en $C_1$ à $C_6$,
(6) un phényle,
(7) un alkyle en $C_1$ à $C_6$-carbonyle,
(8) un di-(alkyle en $C_1$ à $C_6$)-amino,
(9) un phénoxy,
(10) un méthylènedioxy,
(11) un 2,3-furanyle, ou
(12) un 2,3-thiényle, ou

un de leurs sels pharmaceutiquement acceptables.

**2.** Composé selon la revendication 1, dans lequel
R et $R^1$ représentent indépendamment un alkyle en $C_1$ à $C_6$; et
$X_6$ est un carboxy ou un carboxy-alkyle en $C_1$ à $C_6$.

**3.** Composé de la revendication 2, dans lequel:
M représente un alkyle en $C_1$ à $C_3$ ou un allyle; et
$X_6$ représente un hydrogène, un alkyle en $C_1$ à $C_6$, un 3,4-méthylènedioxy ou un phényle.

**4.** Composé de la revendication 3, dans lequel:
R est un éthyle, et
$R^1$ est un méthyle ou un éthyle.

**5.** Composé de la revendication 4, dans lequel:
R et $R^1$ représentent un éthyle;
M est un n-propyle;
$X_6$ est un 4-carboxyméthyle; et
$X_6$ est un 4-méthyle ou un 3,4-méthylènedioxy.

**6.** Composition pharmaceutique pour l'inhibition de l'élastase de leucocytes humains qui comprend une quantité non toxique thérapeutiquement efficace d'un composé de la revendication 1 et un support pharmaceutiquement acceptable.

**7.** Composition de la revendication 6 dans laquelle:
R représente un éthyle,
$R^1$ représente un méthyle ou un éthyle;
M est un alkyle en $C_1$ à $C_3$ ou un allyle;
$X_6$ représente un carboxy ou un carboxy-alkyle en $C_1$ à $C_6$; et
$X_6$ représente un hydrogène, un alkyle en $C_1$ à $C_6$, un 3,4-méthylènedioxy ou un phényle.

**8.** Composition de la revendication 7, dans laquelle:
$R^1$ représente un éthyle;
M représente un n-propyle;
$X_6$ représente un 4-carboxyméthyle; et
$X_6$ représente un 4-méthyle ou un 3,4-méthylènedioxy.

**9.** Procédé de préparation des composés de la revendication 1 dans lequel:
(1) on fait réagir un composé de formule (B) ci-dessus

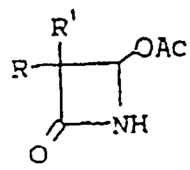

(B)

avec un composé de formule (C)

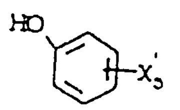

(C)

dans laquelle
$X_{5'}$ représente

(1) un hydrogène,
(2) un alkyle en $C_1$ à $C_6$
(3) un halo-alkyle en $C_1$ à $C_6$,
(4) un alcényle en $C_2$ à $C_6$,
(5) un alcynyle en $C_2$ à $C_6$,
(6) un alcoxy en $C_1$ à $C_6$-carbonyle,
(7) un alcoxy en $C_1$ à $C_6$-carbonyl-alkyle en $C_1$ à $C_6$,
(8) un alcoxy en $C_1$ à $C_6$-carbonyl-alkyle en $C_1$ à $C_6$-carbonyle,
(9) un alcoxy en $C_1$ à $C_6$-carbonyle-alkyle en C1 en $C_1$ à $C_6$-carbonylamino,
(10) un alcoxy en $C_1$ à $C_6$-carbonyle-alcényle en $C_2$ à $C_6$,
(11) un hydroxyalkyle,
(12) un alkyle en $C_1$ à $C_6$-carbonyle,
(13) un alkyle en $C_1$ à $C_6$-carbonylamino, ou
(14) un di-(alkyle en $C_1$ à $C_6$)amino-alkyle en $C_1$ à $C_6$

dans des conditions basiques pour donner un composé de formule (D)

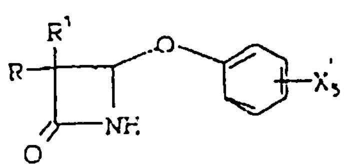

(D)

et (2) on fait réagir le composé (D) avec un composé de formule (E)

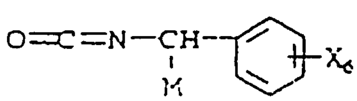

(E)

dans des conditions basiques, et facultativement on transforme $X_{5'}$ en $X_5$, pour donner le composé de formule (A)

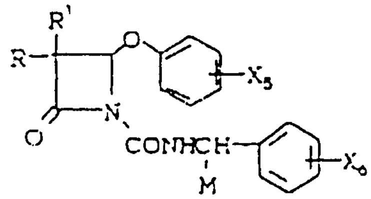

(A).

**10.** Procédé de la revendication 9, dans lequel:
L'étape (1) est conduite en présence d'un hydroxyde de métal alcalin;
L'étape (2) est conduite en présence d'une tri(alkyl en $C_1$ a $C_6$) amine; et
la transformation de $X_{5'}$ en $X_5$ s'effectue en présence d'un acide fort.

**Revendications pour les Etats contractants suivants : ES, GR**

**1.** Procédé de préparation d'un composé de formule (I)

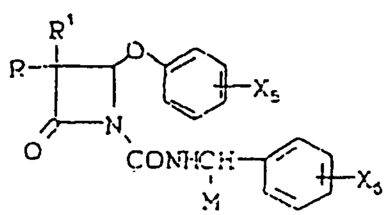

(I)

dans laquelle:
R et $R^1$ représentent indépendamment un alkyle en $C_1$ à $C_6$ ou un alcoxy en $C_1$ à $C_6$-alkyle en $C_1$ à $C_6$;
M représente
(1) un hydrogène,
(2) un alkyle en $C_1$ à $C_6$,
(3) un alcényle en $C_2$ à $C_6$ ou,
(4) un alcoxy en $C_1$ à $C_6$-alkyle en $C_1$ à $C_6$;
$X_5$ représente
(1) un hydrogène,
(2) un alkyle en $C_1$ à $C_6$,
(3) un halo-alkyle en $C_1$ à $C_6$,
(4) un alcényle en $C_2$ à $C_6$,
(5) un alcynyle en $C_2$ à $C_6$,
(6) un carboxy,
(7) un carboxy-alkyle en $C_1$ à $C_6$,
(8) un carboxy-alkyle en $C_1$ à $C_6$-carbonyle,
(9) un carboxy-alkyle en $C_1$ à $C_6$-carbonylamino,
(10) un carboxy-alcényle en $C_2$ à $C_6$,
(11) un hydroxy-alkyle en $C_1$ à $C_6$,
(12) un alkyle en $C_1$ à $C_6$-carbonyle,
(13) un alkyle en $C_1$ à $C_6$-carbonylamino, ou
(14) un di-(alkyle en $C_1$ à $C_6$) amino-alkyle en $C_1$ à $C_6$; et
$X_5$ représente
(1) un hydrogène,
(2) un alkyle en $C_1$ à $C_6$,
(3) un halo,
(4) un carboxy,
(5) un alcoxy en $C_1$ à $C_6$,

(6) un phényle,
(7) un alkyle en $C_1$ à $C_6$-carbonyle,
(8) un di-(alkyle en $C_1$ à $C_6$)-amino,
(9) un phénoxy,
(10) un méthylènedioxy,
(11) un 2,3-furanyle, ou
(12) un 2,3-thiényle, ou

un de leurs sels pharmaceutiquement acceptables,dans lequel

(1) on fait réagir un composé de formule (B) ci-dessus

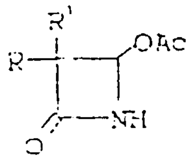

(B)

avec un composé de formule (C)

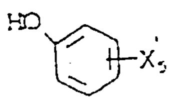

(C)

dans laquelle

$X_{5'}$ représente

(1) un hydrogène,
(2) un alkyle en $C_1$ à $C_6$
(3) un halo-alkyle en $C_1$ à $C_6$,
(4) un alcényle en $C_2$ à $C_6$,
(5) un alcynyle en $C_2$ à $C_6$,
(6) un alcoxy en $C_1$ à $C_6$-carbonyle,
(7) un alcoxy en $C_1$ à $C_6$-carbonyl-alkyle en $C_1$ à $C_6$,
(8) un alcoxy en $C_1$ à $C_6$-carbonyl-alkyle en $C_1$ à $C_6$-carbonyle,
(9) un alcoxy en $C_1$ à $C_6$-carbonyle-alkyle en C1 en $C_1$ à $C_6$-carbonylamino,
(10) un alcoxy en $C_1$ à $C_6$-carbonyle-alcényle en $C_2$ à $C_6$,
(11) un hydroxyalkyle,
(12) un alkyle en $C_1$ à $C_6$-carbonyle,
(13) un alkyle en $C_1$ à $C_6$-carbonylamino, ou
(14) un di-(alkyle en $C_1$ à $C_6$)amino-alkyle en $C_1$ à $C_6$

dans des conditions basiques pour donner un composé de formule (D)

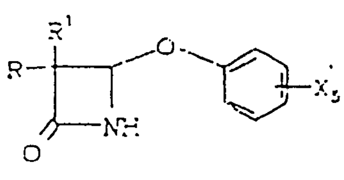

(D)

et (2) on fait réagir le composé (D) avec un composé de formule (E)

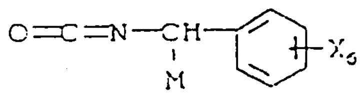

(E)

dans des conditions basiques, et facultativement on transforme $X_{5'}$ en $X_5$, pour donner le composé de formule (A)

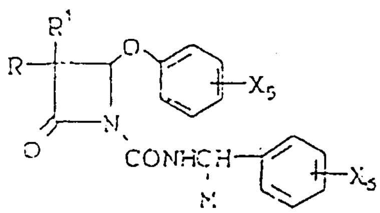

(A)

**2.** Procédé de la revendication 1, dans lequel
R et $R^1$ représentent indépendamment un alkyle en $C_1$ à $C_6$; et
$X_5$ est un carboxy ou un carboxy-alkyle en $C_1$ à $C_6$.

**3.** Procédé de la revendication 2, dans lequel:
M représente un alkyle en $C_1$ à $C_3$ ou un allyle; et
$X_5$ représente un hydrogène, un alkyle en $C_1$ à $C_6$, un 3,4-méthylènedioxy ou un phényle.

**4.** Procédé de la revendication 3, dans lequel:
R est un éthyle, et
$R^1$ est un méthyle ou un éthyle.

**5.** Procédé de la revendication 4, dans lequel:
R et $R^1$ représentent un éthyle;
M est un n-propyle;
$X_5$ est un 4-carboxyméthyle; et
$X_5$ est un 4-méthyle ou un 3,4-méthylènedioxy.

**6.** Procédé de la revendication 1, dans lequel:
L'étape (1) est conduite en présence d'un hydroxyde de métal alcalin;
L'étape (2) est conduite en présence d'une tri(alkyle en $C_1$ à $C_6$) amine; et
la transformation de $X_{5'}$ en $X_5$ s'effectue en présence d'un acide fort.